# EUROPEAN PATENT APPLICATION

(11) **EP 2 768 010 A2**
(43) Date of publication of application: **20.08.2014**
(21) Application number: 14153599.7
(22) Date of filing: 03.02.2014
(51) Int. Cl.: H01J 35/08, H01J 35/14, H01J 35/16

(54) **Radiation tube and radiation imaging system using the tube**

(30) Priority: 19.02.2013 JP 2013029691
(71) Applicant: Canon Kabushiki Kaisha, Tokyo 146-8501 (JP)
(72) Inventor: Kazushige, Utsumi, Tokyo Tokyo 146-8501 (JP); Kazuyuki, Ueda, Tokyo Tokyo 146-8501 (JP)
(74) Representative: TBK

(57) **Abstract**

A radiation tube includes a cathode unit including an electron source, and an anode unit including a target and a shield member arranged around the target. The target is to be irradiated with electrons emitted from the electron source to emit radiation. The cathode unit and the anode unit are joined to each other via a plurality of spacers.

## Description

### BACKGROUND OF THE INVENTION

### Field of the Invention

The present invention generally relates to radiation imaging, and in particular it relates to a radiation tube applicable to X-ray imaging or the like in the field of medical equipment and the field of industrial equipment. An embodiment of the present invention relates to a radiation imaging system using the radiation tube.

### Description of the Related Art

Generally, in a radiation tube including a control electrode, the trajectory of an electron beam emitted from an electron source is controlled by the control electrode. The electron beam, whose trajectory is controlled by the control electrode, is caused to collide with a target, whereby radiation is generated from the target. An example of this structure and process is described in Japanese Patent Application Laid-Open No. 2009-245727. In the same field of endeavor, Japanese Patent Application Laid-Open No. 2009-205992 discusses a multi-X-ray generation apparatus in which a shield member is arranged behind a transmission type target. In this manner, unnecessary radiation and reflected electrons headed backwards are blocked, and an improvement in heat radiation characteristics from the target is achieved. Japanese Patent Application Laid-Open No. 2012-79449 discusses an X-ray tube manufacturing method that employs a jig configured to directly connect an anode unit and a cathode unit to each other before they are separately fixed to an envelope. With this manufacturing method, the anode unit and the cathode unit can be aligned with each other with high precision.

In a radiation tube having the above-mentioned shield member, in order to cause an electron to collide with the target at a desired position without causing it to collide with the shield member, it is necessary to perform positioning of the control electrode system and the target with high precision.

Usually, a plurality of electrodes constituting the control electrode system are arranged such that the centers of the openings of the electrodes through which electrons pass are aligned in the same axis. The center of gravity of an electron beam, which is an aggregation of electrons, extends along this axis and passes through the openings of the control electrode system and the opening of the shield member before the target is irradiated with the electron beam. Accordingly, it is necessary to accurately perform positioning of the center axis of the openings of the control electrode system (hereinafter referred to as the opening axis) and the center axis of the opening of the shield member (hereinafter referred to as the opening axis).

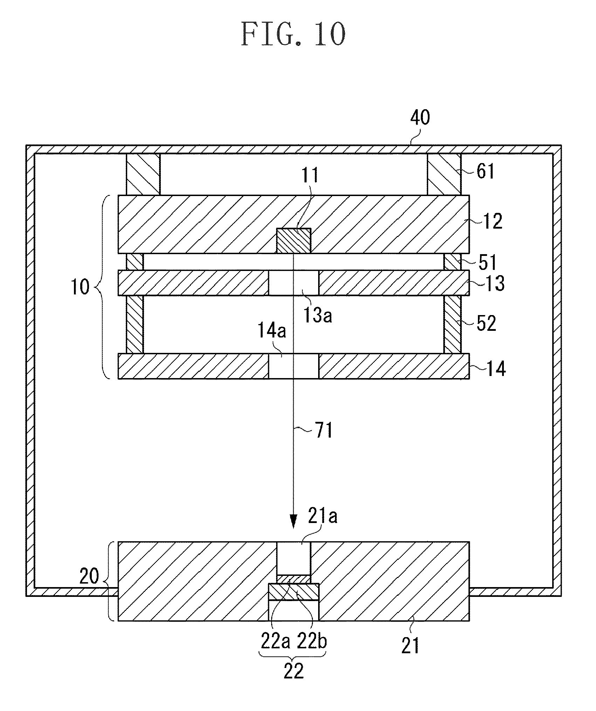

In a conventional radiation tube, however, as illustrated in Fig. 10, a cathode unit 10 including an electron source 11 and an anode unit 20 including a target 22 and a shield member 21 are separately fixed to an envelope 40. Specifically, in Fig. 10, a cathode unit 10, an electron source 11, an extraction electrode 13, and a lens electrode 14 are mounted to an envelope 40 by support members 61 via a retaining member 12. An anode unit 20 includes a target 22 and a shield member 21, and is mounted to an opening provided in one wall surface of the envelope 40 to form a part of the wall surface of the envelop 40.

In such a configuration where the cathode unit 10 and the anode unit 20 are separately mounted to the envelope 40, the positional relationship between an opening 14a of the lens electrode 14 in the control electrode system and an opening 21a of the shield member 21 depends on the machining precision of the envelope 40. When the envelope 40 undergoes deformation due to a temperature change during the operation of the radiation tube, the precision in the positional relationship between the opening 14a of the lens electrode 14 and the opening 21a of the shield member 21 deteriorates.

### SUMMARY OF THE INVENTION

An aspect of the present invention is directed to a radiation tube with a high positional accuracy, which allows a target and a lens electrode constituting the radiation tube to be accurately aligned during the assembly and prevents the target and the lens from being misaligned after the assembly. Another aspect of the present invention is directed to a radiation imaging system capable of obtaining high-precision images by using the radiation tube.

According to a first aspect of the present invention, there is provided a radiation tube as specified in claims 1 to 14. According to a second aspect of the present invention, there is provided a radiation imaging system as specified in claims 15 and 16.

Further features of the present invention will become apparent from the following description of exemplary embodiments with reference to the attached drawings.

### BRIEF DESCRIPTION OF THE DRAWINGS

Figs. 1A and 1B are schematic sectional views of a radiation tube according to a first exemplary embodiment.
Figs. 2A and 2B are perspective views schematically illustrating a manner in which a cathode unit and an anode unit are joined to each other.
Fig. 3 is a schematic sectional view of a radiation tube according to a second exemplary embodiment.
Fig. 4 is a schematic sectional view of a radiation tube according to a third exemplary embodiment.
Fig. 5 is a schematic sectional view of a radiation tube according to a fourth exemplary embodiment.
Figs. 6A and 6B are schematic diagrams respectively illustrating a perspective view and a cross-sectional view a shape of a columnar spacer according to the fourth exemplary embodiment.
Figs. 7A and 7B are schematic diagrams illustrating a configuration of an anode unit according to the fourth exemplary embodiment.
Figs. 8A and 8B are schematic diagrams illustrating another configuration of the anode unit according to the fourth exemplary embodiment.
Fig. 9 is a block diagram illustrating a radiation imaging system according to an embodiment of the present invention.
Fig. 10 is a schematic sectional view of a conventional radiation tube.

### DESCRIPTION OF THE EMBODIMENTS

Various exemplary embodiments, features, and aspects of the invention will be described in detail below with reference to the drawings.

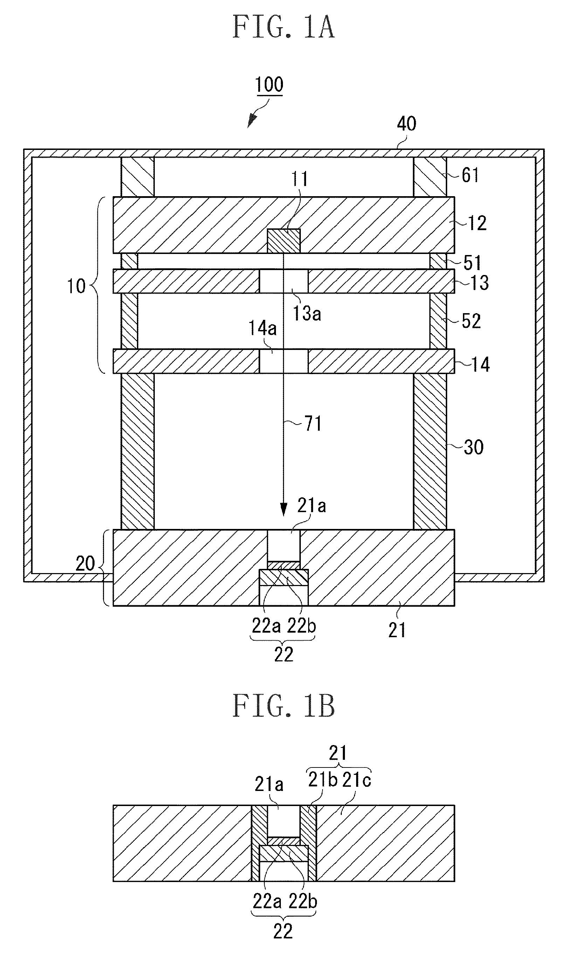

Fig. 1A is a schematic sectional view of a radiation tube 100 according to a first exemplary embodiment of the present invention. A cathode unit 10 includes an electron source 11, an extraction electrode 13, and a lens electrode 14. Electrons emitted from the electron source 11 are drawn out to the anode unit 20 side by the extraction electrode 13. The electrons are converged by the lens electrode 14 to form an electron beam, and then caused to collide with a target 22. The target 22 is a transmission type target, where electrons impinge on a incidence surface of the target and radiation emitted on the surface side opposite to the surface that is irradiated with the electrons is extracted to the outside of the radiation tube 100. The electron source 11 is retained by a retaining member 12. The retaining member 12 and the extraction electrode 13 are joined to each other by electrical insulating spacers 51. The extraction electrode 13 and the lens electrode 14 are joined to each other by electrical insulating spacers 52. The retaining member 12 is mounted (or attached) to an envelope 40 by support members 61.

The electron source 11 may be a thermal electron source consisting of a combination of an electron emitting material and a heater, or may be a cold cathode such as a field emitter. The extraction electrode 13 includes an opening 13a for allowing passage of an electron beam 71 emitted from the electron source 11. The lens electrode 14 includes an opening 14a for allowing passage of the electron beam 71 that has passed through the opening 13a of the extraction electrode 13. As the material for the retaining member 12, the extraction electrode 13, and the lens electrode 14, molybdenum is generally preferred to be used, which is a high-melting-point metal with low vacuum degassing. To insulate the retaining member 12 from the electron source 11, an insulation layer such as an alumina layer is provided between the retaining member 12 and the electron source 11. As the material for the spacers 51 and 52, which are used to join the retaining member 12 and the extraction electrode 13, and the extraction electrode 13 and the lens electrode 14, respectively, an insulation material such as alumina is used.

An anode unit 20 includes the target 22 and a shield member 21 that is arranged around the target 22. The target 22 is composed of a target film 22a and a target substrate 22b that is coated with the target film 22a. As the material for the target film 22a, tungsten or molybdenum is used, and the thickness of the target film 22a ranges approximately from 0.1 µm to 20 µm. The target film 22a is irradiated with an electron beam of high energy density. It is, therefore, desirable that the target substrate 22b has high heat conductivity to enhance the heat radiation property. For example, a diamond substrate with a thickness of 0.3 mm to 5 mm is used. Further, to ensure the required positional precision of the target 22, it is desirable that the target substrate 22b has a high rigid structure made from a high rigid material.

Among the components of radiation and reflected electrons generated from the target 22, the shield member 21 shields those headed backwards, i.e., headed for the cathode unit 10, and also radiates the heat generated from the target 22 to the outside of the radiation tube 100. As a material having a radiation shielding function, oxygen-free copper is preferably used. Further, as illustrated in Fig. 1B, on the inner peripheral side, which is in contact with the target 22, it is desirable to arrange a tungsten layer 21b having a higher radiation insulating function than copper. Further, on the outer periphery of the tungsten layer 21b, a copper layer 21c having a higher heat conductivity than tungsten is provided to maintain the heat radiating function. The anode unit 20 forms a part of the wall surface of the envelope 40, and, therefore, the joint between the tungsten layer 21b and the copper layer 21c requires airtightness enough to maintain the vacuum inside the envelope 40. Thus, considering the required airtightness and heat conductivity, it is desirable to join the tungsten layer 21b and the copper layer 21c by silver brazing.

The anode unit 20 is mounted to an opening provided in the envelope 40, and the cathode unit 10 is joined to the envelope 40 by the support members 61. If the envelope 40 is made from a conductive material, the support members 61 are made from an insulating material. As the conductive material for the envelope 40, SUS304 is preferably used, and, as the insulating material, alumina is preferably used. As the material for the insulating support members 61, alumina is also preferably used. If the envelope 40 is made from a conductive material, the envelope 40 and the anode unit 20 have the same electrical potential. Usually, the target 22 is set to the grounding potential via the shield member 21. Thus, as in the present exemplary embodiment, when the anode unit 20 is mounted to the opening of the envelope 40, and the envelope 40 is made from a conductive material, the target 22 can be set to the grounding potential by grounding the envelope 40. The support members 61 may be mounted to the envelope 40 by being fastened to the inner wall with screws, or by welding. Further, by providing unevenness on the side surfaces of the insulating support members 61 in the longitudinal direction, the support members 61 have a creeping distance larger than their length, which is desirable from the viewpoint of preventing creeping discharge.

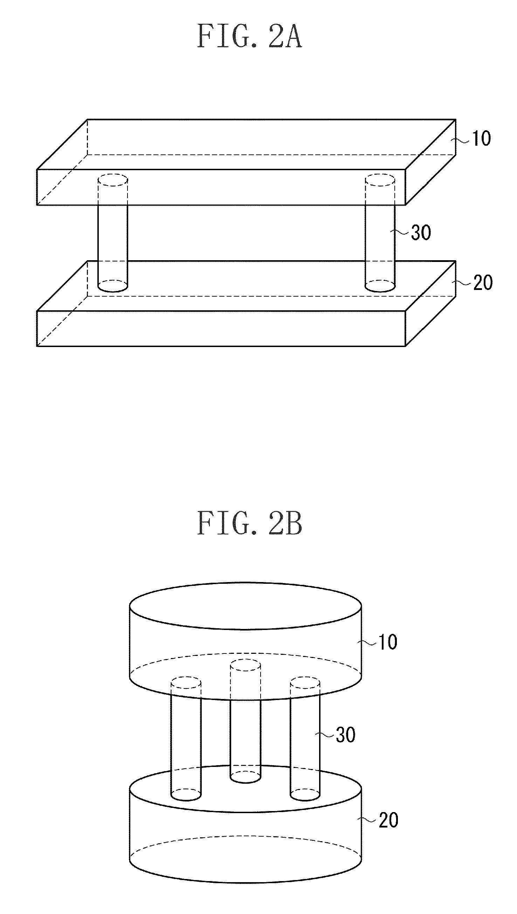

In the present exemplary embodiment, the cathode unit 10 and the anode unit 20 are joined to each other through columnar spacers 30. As illustrated in Figs. 2A and 2B, the cathode unit 10 and the anode unit 20 are joined together through a plurality of the spacers 30. For the anode unit 20, the spacers 30 are mounted to the shield member 21.

As illustrated in Fig. 2A, when the cathode unit 10 and the anode unit 20 are formed as rectangular plates, the spacers 30 are arranged at both end portions of the cathode unit 10 and of the anode unit 20, with the location of the electron source 11 at the center. Further, as illustrated in Fig. 2B, the cathode unit 10 and the anode unit 20 are formed as discs, three spacers 30 are used, and the spacers 30 are arranged at the apexes of a triangle including the electron source 11 and the target 22 at its inner center.

In mounting the spacers 30, two or more reference points are provided on the shield member 21 of the anode unit 20, and reference points corresponding to the above-mentioned reference points are provided on one of the retaining member 12, the extraction electrode 13, and the lens electrode 14 of the cathode unit 10. The reference points of the anode unit 20 and the reference points of the cathode unit 10 are provided in pairs, and the respective pairs are joined together by the respective spacers 30 provided in the same number as the pairs. The positional relationship between the shield member 21 and the target 22, and the positional relationship between the retaining member 12, the extraction electrode 13, or the lens electrode 14, and the electron source 11 are guaranteed by machining precision and assembly precision. The optical axis of the electron beam 71 emitted from the electron source 11 is greatly affected by the position of the lens electrode 14, and, therefore, it is desirable to set the reference points of the cathode unit 10 on the lens electrode 14. For this reason, it is beneficial to join the spacers 30 to the lens electrode 14.

In the present exemplary embodiment, only one electron source 11 is provided, the distance between the spacers 30 is small, and the influence of the thermal deformation of the envelope 40 may be considered low. Thus, the cathode unit 10, the anode unit 20, and the spacers 30 are joined and fixed by silver brazing, adhesive or the like. The spacers 30 are made from an insulating material, and it is desirable to use insulating ceramic, specifically, alumina. By providing unevenness on the side surfaces of the spacers 30 in the longitudinal direction, the creeping distance is increased,
which is desirable from the viewpoint of preventing creeping discharge.

According to the present exemplary embodiment, the cathode unit 10 and the anode unit 20 can be mounted to the envelope 40 in a state that they are joined together through the spacers 30. Thus, alignment between the opening axis of the lens electrode 14 and the opening axis of the shield member 21 can be performed at a minimum distance, thereby realizing high-precision alignment. In order not to affect the positional relationship between the cathode unit 10 and the anode unit 20 already joined to each other, when mounting them to the envelope 40, a method such as laser welding, which causes less distortion, is used to join the envelope 40 and the anode unit 20. If the shield member 21 and the end portions of the envelope 40 are made from materials that cannot be welded together, it is necessary to previously join a material that allows welding. For example, if the envelope 40 is made from alumina, a Kovar material is previously joined to the opening end portion of the envelope 40 by silver brazing.

The radiation tube 100 according to the present exemplary embodiment provides high-precision alignment during the assembly. Even if a positional deviation occurs in the opening 21a of the shield member 21, the cathode unit 10 follows the positional deviation of the anode unit 20 due to the spacers 30. Thus, a deviation in the positional relationship between the opening axis of the shield member 21 and the opening axis of the lens electrode 14 caused as a result of such a positional deviation, can be suppressed smaller than the positional deviation of the opening 21a.

In the present exemplary embodiment described above, the anode unit 20 is mounted to the opening of the envelope 40 and the cathode unit 10 is mounted to the envelope 40 via the insulating support members 61. However, it is also possible to use a reversed mounting arrangement. That is, it is also possible to mount the cathode unit 10 to an opening provided in the envelope 40, and to join the anode unit 20 to the envelope 40 via support members. In this case, if the envelope 40 is a conductive member, the anode unit 20 is joined to the envelope 40 through insulating support members.

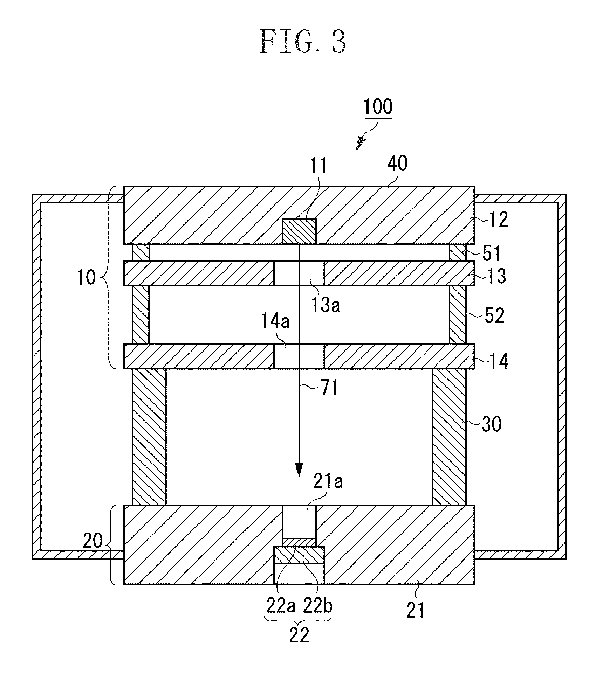

Fig. 3 is a schematic sectional view of a radiation tube according to a second exemplary embodiment of the present invention. The second exemplary embodiment differs from the first exemplary embodiment in that each of the cathode unit 10 and the anode unit 20 forms a part of the wall surface of the envelope 40. That is, openings are respectively provided in two opposite wall surfaces of the envelope 40. The cathode unit 10 is mounted to one opening and the anode unit 20 is mounted to the other opening. Apart from this, the second exemplary embodiment is the same as the first exemplary embodiment. In the second exemplary embodiment, it is also possible to use either of a conductive material and an insulating material as the material for the envelope 40. However, if the envelope 40 is made from a conductive material, at least one of the cathode unit 10 and the anode unit 20 is joined to the envelope 40 through insulating members.

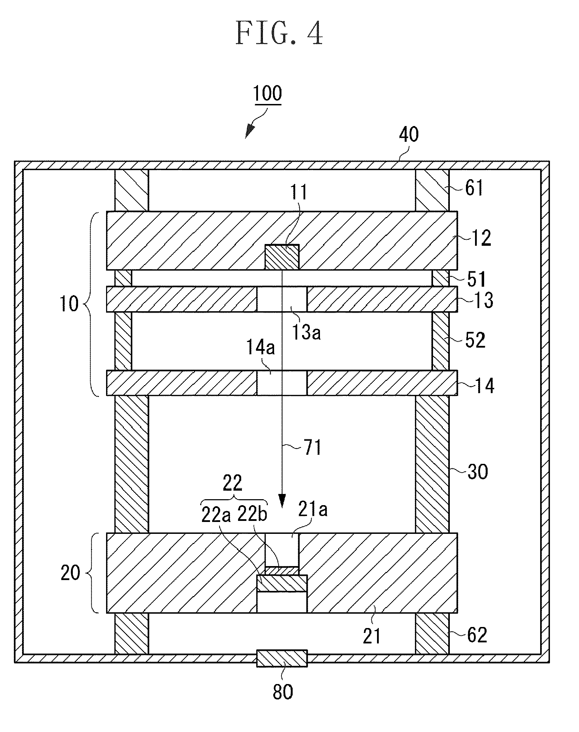

Fig. 4 is a schematic sectional view of a radiation tube according to a third exemplary embodiment of the present invention. The third exemplary embodiment differs from the first exemplary embodiment in that both the cathode unit 10 and the anode unit 20 are mounted to the envelope 40 through the support members 61 and 62. Apart from this, the third exemplary embodiment is the same as the first exemplary embodiment. In the third exemplary embodiment, an opening is previously provided in the envelope 40, and the cathode unit 10 and the anode unit 20, which have been joined together through the spacers 30, are accommodated in the envelope 40 via the opening. Then, the opening is hermetically sealed by the corresponding lid, so that the inside of the envelope 40 is kept airtight. Any method may be used to join the support members 61 and 62 to the envelope 40 so long as it does not affect the positional relationship between the cathode unit 10 and the anode unit 20. For example, the support members 61 and 62 may be joined to the inner wall of the envelope 40 by screws, or to the envelop 40 by welding. In the third exemplary embodiment, either of a conductive member and an insulating member can be used as the envelope 40, as in the first exemplary embodiment. However, if the envelope 40 is a conductive member, the support members 61 and 62 needs to be insulating members.

In the third exemplary embodiment, the anode unit 20 is inside the envelope 40, and it is, therefore, necessary to provide a window 80 that allows the radiation generated from the target 22 to be transmitted to the outside of the envelope 40. More specifically, in the portion of the envelope 40 that faces the opening 21a of the shield member 21, a window made from, for example, beryllium is provided so that compatibility between the transmission of radiation and the ensuring of vacuum airtightness is achieved.

In the first through third exemplary embodiments, only one electron source is provided. However, in a fourth exemplary embodiment of the present invention, a plurality of electron sources is provided. For the plurality of electron sources, it is possible that the number of extraction electrode openings and of lens electrode openings is one. However, it is desirable that the number is equal to the number of electron sources. If the number is one, the electrons emitted from the plurality of electron sources are collectively controlled. The target is arranged corresponding to the opening of the extraction electrode and that of the lens electrode. If a plurality of electron sources are provided, the number of extraction electrode openings and of lens electrode openings is one, and one target is provided, the configuration is such that the one opening of the lens electrode and the one opening of the shield member are aligned with each other, as in the first through third exemplary embodiments.

In the fourth exemplary embodiment, a configuration will be described, in which an extraction electrode with a plurality of openings and a lens electrode with a plurality of openings are provided, each of the openings of the electrodes corresponds to each of a plurality of electron sources, and each of a plurality of targets is irradiated with electrons. The components other than those described below are similar to the components in the first through third exemplary embodiments, and the description thereof will be omitted.

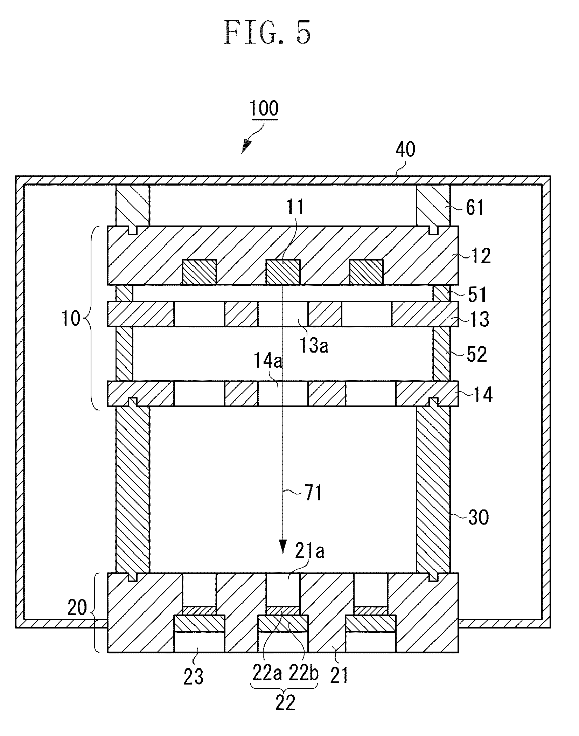

As an example, Fig. 5 illustrates a configuration in which three electron sources 11 and three targets 22 are provided. In this case, three openings 13a of the extraction electrode 13 and three openings 14a of the lens electrode 14 are also formed corresponding to the electron sources 11 and the targets 22. The distance between two adjacent electron sources 11 ranges approximately from 5 mm to 50 mm. The electron sources 11 and the openings 13a and 14a are arranged in rows, and the spacers 30 are arranged at both end portions in their arranging direction. The number of the electron sources 11 and the number of the targets 22 is not restricted to three but ranges usually from 10 to 50 depending on the application. Further, the number of the spacers 30 is not restricted to two but may also be three or more. However, if the number is four or more, the distance between the cathode unit 10 and the anode unit 20 may not be uniquely determined. Thus, it is desirable that the number is two.

Joining the cathode unit 10 and the anode unit 20 to the spacers 30 is not performed by silver brazing, adhesive or the like but performed by concave-convex fitting and application of a force to pinch the fitting portions. This is due to the fact that there is a plurality of electron sources 11 and that the distance between the two spacers 30 is large, resulting in a great influence of heat changes in the envelope 40.

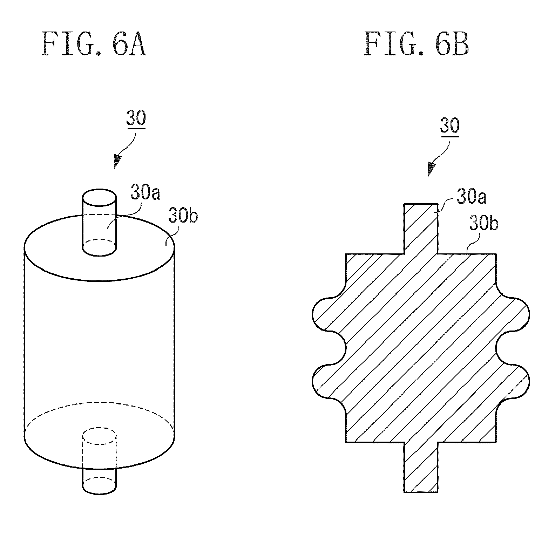

As illustrated in Fig. 6A, each of the spacers 30 used in the fourth exemplary embodiment has a columnar shape with protrusions 30a at both ends and flat portions 30b around the protrusions 30a. The surface-to-surface distance between the flat portions 30a (the height of the column) ranges from 5 mm to 30 mm. The flat portions 30b are respectively in contact with the cathode unit 10 and the anode unit 20, so that the surface-to-surface distance between the flat portions 30b determines the distance between the cathode unit 10 and the anode unit 20. As illustrated in Fig. 6A, the side surface of the spacer 30 may have a flat shape, or, as illustrated in Fig. 6B, it may have a concave-convex shape in the longitudinal direction. Thus, by providing unevenness on the side surface of the spacer 30, the creeping distance from one flat portion 30b to the other flat portion 30b is larger than the surface-to-surface distance, which is desirable from the viewpoint of preventing creeping discharge.

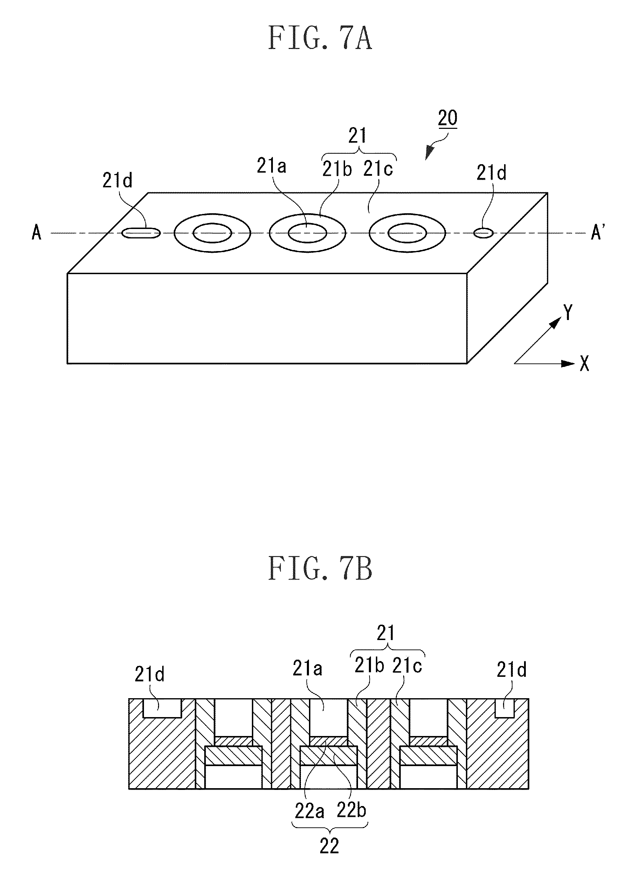

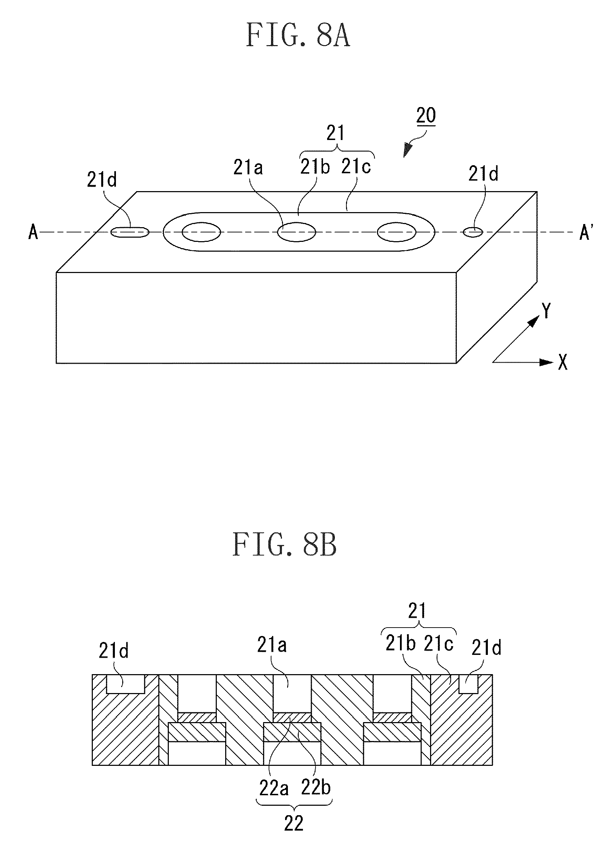

The protrusions 30a of the spacer 30 are fitted into recesses each provided in the cathode unit 10 and the anode unit 20. More specifically, as illustrated in Figs. 7A and 7B and Figs. 8A and 8B, recesses 21d corresponding to the protrusions 30a of the spacers 30 are provided in the anode unit 20. Similarly, recesses are provided in the cathode unit 10. Recesses similar to the recesses 21d illustrated in Figs. 7A and 7B are provided in one of the retaining member 12, the extraction electrode 13, and the lens electrode 14. Figs. 7B and 8B are schematic vertical sectional views taken along the line A-A' of Figs. 7A and 8A. These recesses 21d correspond to the above-mentioned reference points. If the recesses 21d are provided in the lens electrode 14, they are respectively provided at both end portions thereof in the arranging direction of the openings 14a. For the shield member 21, the recesses 21d are respectively provided at both end portions thereof in the arranging direction of the openings 21a.

The protrusions 30a of the spacers 30 are formed to be fitted into the recesses serving as the reference points provided in the cathode unit 10 and the anode unit 20, leaving a gap of 0.01 mm to 0.1 mm. As a result, the cathode unit 10 and the anode unit 20 can be joined together with a positional precision of 0.01 mm to 0.1 mm.

The flat portions 30b of the spacers 30 are to be brought into contact with the flat portions provided around the recesses serving as the reference points provided in the cathode unit 10 and the anode unit 20, so that the parallelism between the cathode unit 10 and the anode unit 20 can be maintained at a level not higher than a fixed level. The parallelism is set to be 0.02 mm to 0.1 mm. The parallelism can be expressed as the parallelism of the surface of the lens electrode 14 on the anode unit 20 side when the surface of the anode unit 20 on the cathode unit 10 side is used as the reference surface. The gaps between the recesses serving as the reference points provided in the cathode unit 10 and the anode unit 20, and the protrusions 30a of the spacers 30 can serve as play for allowing shape changes during the operation of the radiation tube 100. In some cases, the size of such gaps is insufficient even if it is 0.1 mm, and shearing stress is applied to the spacers 30. Taking into account such cases, as illustrated in Figs. 7A and 7B and Figs. 8A and 8B, one of the two recesses 21d serving as the reference points provided in the anode unit 20 (the one on the left-hand side in Figs. 7A and 7B and Figs. 8A and 8B) may be formed as an elongated hole to secure the play for allowing shape changes. In this case, the reference point in the form of an elongated hole cannot fix the positions of the openings 21a of the shield member 21. It is possible, however, to prevent the positional deviation in the rotational direction. That is, in the case of Figs. 7A and 7B and Figs. 8A and 8B, it is not possible to fix the positions of the openings 21a in the X-direction. However, there is little play in the Y-direction, so that it is possible to prevent rotation of the anode unit 20 within the XY-plane.

Further, in the fourth exemplary embodiment, it is desirable that the anode unit 20 includes the shield member 21 formed of the tungsten layer 21b and the copper layer 21c, as in the above-described exemplary embodiments. As illustrated in Figs. 7A and 7B, it is possible to form the tungsten layer 21b on the inner peripheral side of each of the openings 21a, and to form the copper layer 21c on the outer side thereof. As illustrated in Figs. 8A and 8B, it is possible to use a configuration in which the tungsten layer 21b forming the inner periphery of each of the openings 21a is continuous throughout all the openings 21a.

In the fourth exemplary embodiment, the cathode unit 10 is joined to the envelope 40 through the support members 61. It is desirable that each of the support members 61 has a columnar shape having a protrusion at one end thereof and the protrusion is fitted into a recess provided in the retaining member 12. In this case, by leaving a gap of 0.5 mm to 2 mm in the fitting portion between the support members 61 and the cathode unit 10, the required play can be secured.

A case will be described where the multi-radiation source according to the fourth exemplary embodiment is applied to a radiation imaging system for mammography that performs tomosynthesis imaging. In tomosynthesis imaging, radiation is applied from different angles to the same region on the irradiation surface of a subject. That is, radiation is applied at angles in a number equal to the number of radiation sources each formed of a pair of an electron source and a target. In this case, front openings 23 of the shield member 21 are formed such that the radiations emitted from the respective targets 22 are applied to the same region on the irradiation surface. That is, the columnar openings 23 are formed such that the direction of the center axis thereof differs according to the arrangement positions of the openings 23. For example, when the center axis of the opening 23 arranged at the center of the anode unit 20 matches the axis along which an electron beam passes, the closer the openings 23 are formed to both end portions of the anode unit 20, the further the center axis is inclined toward the center side. As a result, the center axes of cone-shaped radiation beams emitted from the respective radiation sources can cross each other at a single point on the irradiation surface.

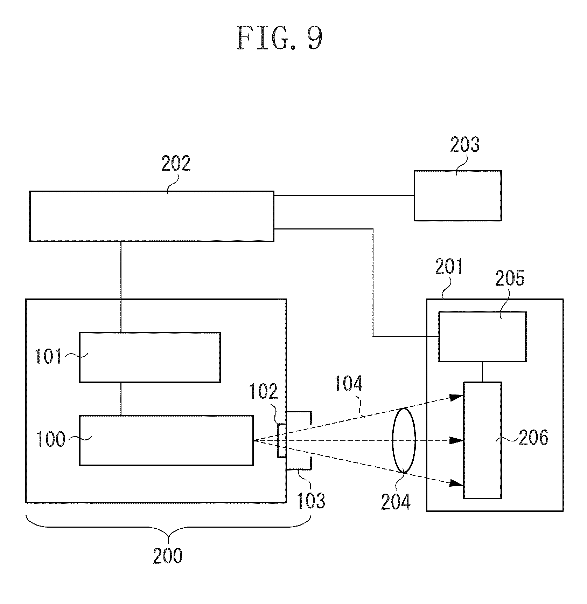

Referring to Fig. 9, a radiation imaging system according to a fifth exemplary embodiment of the present invention will be described.

As illustrated in Fig. 9, the radiation tube 100 according to the first through fourth exemplary embodiments is incorporated into a radiation generation apparatus 200 together with a radiation drive circuit 101 for controlling the drive of the radiation tube 100. Radiation 104 generated by the radiation tube 100 is emitted to the outside of the radiation generation apparatus 200 via a radiation emission window 102. A movable diaphragm unit 103 provided at the radiation emission window 102 portion of the radiation generation apparatus 200 has a function of adjusting the size of the irradiation field of the radiation emitted from the radiation tube 100. As the movable diaphragm unit 103, it is also possible to use the one having an additional function of providing a simulated display of the irradiation field of the radiation by visible light.

A system control apparatus 202 (e.g., a programmed microprocessor or general computer) controls the radiation generation apparatus 200 and a radiation detection apparatus 201 in conjunction with each other. The drive circuit 101 outputs various control signals to the radiation tube 100 under the control of the system control apparatus 202. The control signals allows the radiation emitted from the radiation generation apparatus 200 to be transmitted through a subject 204 and then detected by a detection apparatus 206. The detection apparatus 206 converts the detected radiation to an image signal, and outputs the image signal to a signal processing unit 205. Under the control of the system control apparatus 202, the signal processing unit 205 performs predetermined signal processing on the image signal, and outputs the processed image signal to the system control apparatus 202. Based on the processed image signal, the system control apparatus 202 outputs to a display apparatus 203 a display signal for displaying an image on the display apparatus 203. The display apparatus 203 displays on the display an image based on the display signal as a captured image of the subject 204. A typical example of the radiation is an X-ray, and the radiation tube 100 and the radiation imaging system according to the fifth exemplary embodiment can be utilized as an X-ray generation unit and an X-ray imaging system. The X-ray imaging system can be used for the nondestructive inspection of an industrial product and for the pathological diagnosis of a human body and an animal.

According to the above-described exemplary embodiments, the radiation tube 100 provides high-precision alignment between the control electrode system and the target, and suppresses the positional deviation during the operation, so that high-precision captured images can be obtained.

In the exemplary embodiments of the present invention, the cathode unit and the anode unit are joined together through a plurality of spacers, so that, during the assembly, the cathode unit and the anode unit can be mounted to the envelope in a state where the units have been joined together. More specifically, the radiation tube is assembled in a state where the opening axis of the lens electrode included in the cathode unit and the opening axis of the shield member included in the anode unit are directly aligned with each other, so that high-precision alignment can be maintained. Further, even if a positional deviation occurs in one of the cathode unit and the anode unit due to thermal deformation of the envelope during the operation of the radiation tube, the other unit follows the positional deviation via the spacers, so that a relative positional deviation between the units can be reduced.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

A radiation tube includes a cathode unit including an electron source, and an anode unit including a target and a shield member arranged around the target. The target is to be irradiated with electrons emitted from the electron source to emit radiation. The cathode unit and the anode unit are joined to each other via a plurality of spacers.

## Claims

1. A radiation tube comprising:
a cathode unit including an electron source; and
an anode unit including a target configured to be irradiated with electrons emitted from the electron source to emit radiation, and a shield member arranged around the target,
wherein the cathode unit and the anode unit are joined to each other via a plurality of spacers.

2. The radiation tube according to claim 1, wherein the plurality of spacers has a columnar shape.

3. The radiation tube according to claim 1 or 2, wherein the cathode unit further includes:
a retaining member configured to retain the electron source;
an extraction electrode that has an opening for allowing passage of the electrons emitted from the electron source and is joined to the retaining member; and
a lens electrode that has an opening for allowing passage of the electrons and is joined to the extraction electrode,
wherein the plurality of spacers is joined to the lens electrode and the shield member.

4. The radiation tube according to claim 3, wherein the lens electrode is made from molybdenum.

5. The radiation tube according to claim 3 or 4, wherein the shield member is formed of a tungsten layer that is in contact with the target and located on an inner peripheral side, and a copper layer that is located on an outer periphery of the tungsten layer.

6. The radiation tube according to any preceding claim, wherein the cathode unit includes a plurality of electron sources arranged in a row,
wherein the anode unit includes a plurality of targets arranged in association with the respective plurality of electron sources, and
wherein the plurality of spacers is located outside electron sources arranged at both ends, and outside both ends corresponding to the electron sources arranged at both ends.

7. The radiation tube according to any preceding claim, further comprising an envelope formed of wall surfaces and having an opening in each of two wall surfaces opposite to each other,
wherein the cathode unit is mounted to one opening of the two wall surfaces and the anode unit is mounted to the other opening of the two wall surfaces, and
wherein a vacuum is maintained inside the envelope.

8. The radiation tube according to claim 7, wherein the envelope is electrically insulating.

9. The radiation tube according to any one of claims 1 to 6, further comprising an envelope formed of wall surfaces and having an opening in one wall surface,
wherein one of the cathode unit and the anode unit is mounted on the opening of the wall surface, and
wherein a vacuum is maintained inside the envelope.

10. The radiation tube according to claim 9, wherein the envelope is conductive.

11. The radiation tube according to any one of claims 1 to 6, wherein the cathode unit and the anode unit are accommodated inside an envelope, and
wherein a vacuum is maintained inside the envelope.

12. The radiation tube according to claim 11, wherein the envelope is conductive.

13. The radiation tube according to any preceding claim, wherein the plurality of spacers is made from insulating ceramic and has unevenness on a side surface in a longitudinal direction.

14. The radiation tube according to any preceding claim, wherein the target is a transmission type target.

15. A radiation imaging system comprising:
the radiation tube according to any preceding claim;
a radiation detection apparatus configured to detect radiation that has been emitted from the radiation tube and transmitted through a subject; and
a control apparatus configured to control the radiation tube and the radiation detection apparatus in conjunction with each other.

16. A radiation imaging system for mammography that performs tomosynthesis imaging by applying radiation from different angles to a subject, the radiation imaging system comprising:
the radiation tube according to claim 6;
a radiation detection apparatus configured to detect radiation that has been emitted from the radiation tube and transmitted through a subject; and
a control apparatus configured to control the radiation tube and the radiation detection apparatus in conjunction with each other.
